# EUROPÄISCHE PATENTSCHRIFT

(11) **EP 1 165 535 B1**
(45) Veröffentlichungstag und Bekanntmachung des Hinweises auf die Patenterteilung: **24.07.2002**
(21) Anmeldenummer: 00926765.9
(22) Anmeldetag: 28.03.2000
(51) Int. Cl.: C07D 301/06

(54) **VERFAHREN ZUR HERSTELLUNG VON EPOXIDEN**
METHOD OF PRODUCING EPOXY RESINS
PROCEDE DE PRODUCTION DE RESINES EPOXY

(30) Priorität: 08.04.1999 DE 19915903
(43) Veröffentlichungstag der Anmeldung: 02.01.2002
(73) Patentinhaber: Studiengesellschaft Kohle mbH, 45470 Mülheim an der Ruhr (DE)
(72) Erfinder: LEITNER, Walter, D-45468 Mülheim/Ruhr (DE); LOEKER, Frank, D-47608 Geldern (DE)
(74) Vertreter: von Kreisler, Alek, Dipl.-Chem.
(86) Internationale Anmeldenummer: EP0002719
(87) Internationale Veröffentlichungsnummer: WO0061570

(56) Entgegenhaltungen:
- EP-A- 0 540 009
- GB-A- 970 337
- GB-A- 1 302 143
- US-A- 5 210 336
- BIRNBAUM E R ET AL: "Metalloporphyrin-catalyzed homogeneous oxidation in supercritical carbon dioxide" J. MOL. CATAL. A: CHEM. (JMCCF2,13811169);1999; VOL.139 (1); PP.11-24, XP000929940 Los Alamos National Laboratory;Chemical Science and Technology Division, Los Alamos Catalysis Initiative; Los Alamos; 87545; NM; USA (US)
- PESIRI D R ET AL: "Selective epoxidation in dense phase carbon dioxide" CHEM. COMMUN. (CAMBRIDGE) (CHCOFS,13597345);1998; (9); PP.1015-1016, XP002144695 University of North Carolina;Department of Environmental Sciences and Engineering; Chapel Hill; 27599; NC; USA (US)
- HAAS G R ET AL: "The Diastereoselective Epoxidation of Olefins in Supercritical Carbon Dioxide" TETRAHEDRON LETTERS,NL,ELSEVIER SCIENCE PUBLISHERS, AMSTERDAM, Bd. 39, Nr. 33, 13. August 1998 (1998-08-13), Seiten 5923-5926, XP004126761 ISSN: 0040-4039

## Beschreibung

Die vorliegende Erfindung betrifft die Herstellung von Epoxiden durch Oxidation von Olefinen mittels molekularem Sauerstoff unter Zusatz eines aldehydischen Additivs, dadurch gekennzeichnet, daß die Oxidation in komprimiertem Kohlendioxid als Lösungsmittel durchgeführt wird.

Die Herstellung von Epoxiden durch Oxidation von Olefinen ist ein technisch wichtiger Prozeß von großer wirtschaftlicher Bedeutung (vgl. Römpp-Chemie Lexikon, Georg Thieme Verlag Stuttgart-New York 1997, 10. Auflage, Band 2, 1186). Aus ökologischer und ökonomischer Sicht ist die Verwendung von molekularem Sauerstoff als Oxidans (in Form von reinem Sauerstoff oder von Luftsauerstoff) von besonderem Interesse. Dies gelingt beispielsweise durch Zusatz von aldehydischen Additiven zu Reaktionsmischungen bestehend aus dem Substrat und einem geeigneten flüssigen organischen Lösungsmittel, wobei jedoch in der Regel die Anwesenheit von teuren und aufwendig herzustellenden Übergangsmetallkatalysatoren erforderlich ist (Bull. Chem. Soc. Jpn. 1995, 68, 17-35 und Chem. Commun. 1997, 69-70).

Die Oxidation von Cyclohexen zu Epoxycyclohexan mit molekularem Sauerstoff unter Zusatz eines aldehydischen Additivs in CH₂Cl₂ als Lösungsmittel wurde mit einem einfachen Katalysator beschrieben, der aus Eisenpulver und katalytischen Mengen einer Carbonsäure (bevorzugt Essigsäure) hergestellt werden kann (J. Am. Chem. Soc. 1992, 114, 7913-7914). In der Anmeldung EP 0 540 009 A1 wird beschrieben, daß unter diesen Bedingungen sogar gänzlich auf einen Katalysator verzichtet werden kann. Die Verwendung großer Mengen des ökologisch und toxikologisch bedenklichen Lösungsmittels CH₂Cl₂ stellt jedoch einen erheblichen Nachteil dieser Reaktionen dar. Unsere eigenen Untersuchungen (Beispiel 12 - 15) zeigen, daß CH₂Cl₂ in diesem Prozeß nicht einfach durch andere, weniger bedenkliche Lösungsmittel wie z. B. Toluol ersetzt werden kann, da erheblich niedrigere Ausbeuten und Nebenreaktionen durch Oxidation des Lösungsmittels resultieren. Desweiteren müssen bei der Verwendung vieler herkömmlicher organischer Lösungsmittel die Explosionsgrenzen der Lösungsmittel berücksichtigt werden, wenn molekularer Sauerstoff als Oxidationsmittel dient, wodurch der Partialdruckbereich des Sauerstoffs stark limitiert wird.

Der vorliegenden Anmeldung lag also das Problem zugrunde, ein geeignetes Lösungsmittel für die Epoxidierung von Olefinen mit molekularem Sauerstoff unter Zusatz eines aldehydischen Additivs zu finden, wobei das Lösungsmittel oxidationsstabil, die Explosionsgefahr mindernd sowie toxikologisch und ökologisch unbedenklich sein sollte.

Wir haben gefunden, daß komprimiertes (flüssiges oder überkritisches) Kohlendioxid diese Anforderungen in idealer Weise erfüllt und beschreiben hier ein Verfahren zur Herstellung von Epoxiden der allgemeinen Formel **II** (s. u.) durch Oxidation von Olefinen der allgemeinen Formel **I** (s. u.) mittels molekularem Sauerstoff unter Zusatz eines aldehydischen Additivs der allgemeinen Formel **III** (s. u.), dadurch gekennzeichnet, daß die Oxidation in komprimiertem Kohlendioxid als Lösungsmittel durchgeführt wird.

Als Olefine können Substrate der allgemeinen Formel **I** eingesetzt werden, 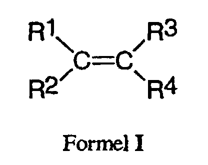 wobei R¹, R², R³ und R⁴ gleich oder verschieden sein können, unabhängig voneinander wählbar sind und ein Wasserstoffatom sein können oder geradkettige, verzweigte, cyclische oder aromatische (C₁-C₂₀)-Kohlenwasserstoffe enthalten können, in denen ein oder mehrere Kohlenstoffe durch Heteroatome ersetzt sein können oder/und die mit halogen-, hydroxy-, alkoxy-, phenoxy-, acyloxy-, acyl-, alkoxycarbonyl- oder phenoxycarbonyl-Gruppen substituiert sein können, wobei die Reste R¹, R², R³ oder R⁴ untereinander zu Ringen oder kondensierten Ringsystemen verbunden sein können. Die Olefine der allgemeinen Formel **I** werden in dem von uns entwickelten Verfahren zu den entsprechenden Epoxiden der allgemeinen Formel **II** oxidiert, 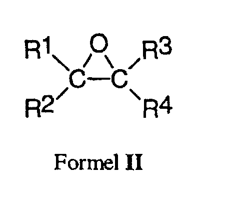 wobei R¹, R², R³ und R⁴ die gleiche Definition wie in Formel **I** besitzen. Dabei wird ein aldehydisches Additiv der allgemeinen Formel **III** zugesetzt, 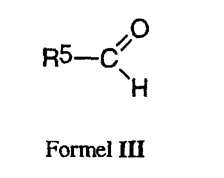 wobei R⁵ ein Wasserstoffatom, eine (C₁-C₁₀)-Alkylgruppe, eine halogen-substituierte (C₁-C₁₀)-Alkylgruppe, eine Arylgruppe, eine halogen-substituierte Arylgruppe, eine Arylalkylgruppe oder eine halogen-substituierte Arylalkylgruppe sein kann.

Als Lösungsmittel dient bei der vorliegenden Erfindung komprimiertes, bevorzugt überkritisches Kohlendioxid (scCO₂, kritische Daten: T_{c} = 30.9 °C, p_{c} = 73.75 bar, ρ_{c} = 0.468 g cm⁻³). Der Gesamtdruck liegt im Bereich von 40 - 500 bar, bevorzugt von 60 - 200 bar.

Die Reaktionstemperatur liegt im Bereich von 0 - 200 °C, bevorzugt zwischen 31 °C und 100 °C. Die Reaktionszeit unterliegt keiner allgemeinen Beschränkung und richtet sich nach dem Verlauf der Reaktion, der mittels geeigneter Analysenmethoden (z. B. GC, NMR, IR) untersucht werden kann. Die Reaktionszeit liegt gewöhnlich im Bereich von 1 bis 48 Stunden.

Die Menge an eingesetztem Aldehyd unterliegt keiner besonderen Beschränkung, beträgt aber gewöhnlich 0.1 - 30 mol, bevorzugt 1 - 10 mol, pro mol Olefin.

Die Menge an eingesetztem Olefin unterliegt keiner besonderen Beschränkung, beträgt aber gewöhnlich 0.1 - 50 mmol pro 100 g CO₂, bevorzugt 0.5 - 10 mmol pro 100 g CO₂. Es ist zu beachten, daß mit Erhöhung der Menge an Olefin auch die Explosionsgefahr nach Zugabe von Sauerstoff steigt.

Der molekulare Sauerstoff kann in reiner Form oder in Form von Gemischen mit anderen Gasen (z. B. als Luftsauerstoff) eingesetzt werden. Der Partialdruck des eingesetzten Sauerstoffs unterliegt keiner besonderen Beschränkung, beträgt aber gewöhnlich 1 - 20 bar, bevorzugt 1 - 5 bar. Dabei liefert die hier beschriebene Verwendung von komprimiertem CO₂ als Lösungsmittel gegenüber vielen herkömmlichen organischen Lösungsmitteln den Vorteil, daß CO₂ als Lösungsmittel oxidationsstabil ist und die Explosionsgefahr mindert. Daher können nach dem Lösen von Olefin und Aldehyd in der als Lösungsmittel benötigten Menge an CO₂ im Unterschied zu vielen herkömmlichen organischen Lösungsmitteln auch höhere Partialdrücke an Sauerstoff gefahrlos angewendet werden.

Das von uns beschriebene Verfahren ist auch mit der Verwendung bekannter Oxidationskatalysatoren kompatibel. Solche Katalysatoren können zur Erhöhung der Reaktionsgeschwindigkeit oder der Steigerung der Selektivität dienen. Die Katalysatoren können in fester, flüssiger oder im Reaktionsmedium gelöster Form eingesetzt werden. Typische Katalysatoren sind ausführlich in Bull. Chem. Soc. Jpn. 1995, 68, 17-35 und EP 0 540 009 A1 beschrieben, das Verfahren ist aber nicht auf die dort erwähnten Katalysatoren beschränkt.

Aufgrund der mit Druck und Temperatur variablen Lösungseigenschaften von überkritischem Kohlendioxid ist ferner bei geeigneter Wahl der äußeren Parameter die Abtrennung von Haupt- (Epoxid), Nebenprodukten (Carbonsäure des aldehydischen Additivs, weitere Oxidationsprodukte des Olefins) und den nicht umgesetzten Edukten (Olefin und Aldehyd) aus der Reaktionsmischung möglich. Bekannte Verfahren, in denen scCO₂ zur Stofftrennung genutzt wird, sind in Angew. Chem. Int. Ed. Engl. 1978, 17, 702 und in M. McHugh, V. Krukonis, Supercritical Fluid Extraction, Butterworth Publishers, 1986 beschrieben. Bei zusätzlicher Verwendung eines Katalysators ist auch dessen Abtrennung möglich.

Anhand der folgenden, ausgewählten Beispiele soll die vorliegende Erfindung näher beschrieben werden, wobei die Anwendung des Verfahrens durch die gewählten Beispiele jedoch in keiner Weise limitiert werden soll.

### Beispiel 1 - 4

Cis-Cycloocten (2.50 mmol) wurde zusammen mit dem Additiv Isobutyraldehyd im molaren Verhältnis (C/I) in einen 100 ml Stahlautoklaven gegeben, und CO₂ wurde bis zu einer Dichte (ρ(CO₂) = Einwaage CO₂/Reaktorvolumen) von 0.75 g cm⁻³ mittels Kompressor zugeführt. Anschließend wurde der Druck im Autoklaven mit molekularem Sauerstoff so weit erhöht, daß die O₂-Menge dem gewünschten Partialdruck von 3 bar entsprach. Der Autoklav wurde bis zur Reaktionstemperatur T aufgeheizt, und nach der Reaktionszeit t wurde die Produktzusam-mensetzung mittels GC analysiert. Dabei wurde die Produktverteilung durch Integration der Peakflächen im Gaschromatogramm ermittelt. Der GC-Faktor von Epoxycyclooctan wurde anhand einer Eichmessung ermittelt. Die Identifizierung der Oxidationsprodukte erfolgte mittels GC/MS-Analyse. Die Reaktionsergebnisse sind in Tabelle 1 zusammengestellt.

**Tabelle 1**

| Beispiel Nr. | C/I | T (°C) | t (h) | Umsatz (%)[a] | Selektivität (%)[b] |
|---|---|---|---|---|---|
| 1 | 1:2 | 55-56 | 18 | > 99 | > 99 |
| 2 | 1:2 | 55-56 | 8 | 90 | > 99 |
| 3 | 1:1 | 55-56 | 18 | 2 | > 95 |
| 4 | 1:2 | 25-28[c] | 18 | 14 | > 99 |

| | | | | | |
|---|---|---|---|---|---|
| [a] bezogen auf cis-Cycloocten | | | | | |
| [b]Selektivität = (GC-Flächenanteil des Epoxycyclooctans)/(GC-Flächenanteil aller detektierten Oxidationsprodukte des Olefins). Oxidationsprodukte mit einem Flächenanteil kleiner 0.2 % wurden vernachlässigt. | | | | | |
| [c] in flüssigem CO₂ | | | | | |

### Beispiel 5

Cis-Cycloocten (2.50 mmol) wurde zusammen mit dem Additiv Isobutyraldehyd (5.00 mmol) unter einer Atmosphäre molekularen Sauerstoffs in einen 100 ml Stahlautoklaven gegeben, und CO₂ wurde bis zu einer Dichte (ρ(CO₂) = Einwaage CO₂/Reaktorvolumen) von 0.75 g cm⁻³ mittels Kompressor zugeführt. Der Autoklav wurde bis zu einer Reaktionstemperatur von 55-56 °C aufgeheizt, und nach einer Reaktionszeit von 18 Stunden wurde die Produktzusammensetzung analog Beispiel 1 - 4 analysiert. Der Umsatz an cis-Cycloocten betrug 94 % mit einer Selektivität bezüglich Epoxycyclooctan von > 99 %.

### Beispiel 6 - 10

Das Olefin (2.50 mmol) wurde zusammen mit dem Additiv Isobutyraldehyd (5.00 mmol) in einen Stahlautoklaven gegeben, und CO₂ wurde bis zu einer Dichte (ρ(CO₂) = Einwaage CO₂/Reaktorvolumen) von 0.75 g cm⁻³ mittels Kompressor zugeführt. Anschließend wurde der Druck im Autoklaven mit molekularem Sauerstoff so weit erhöht, daß die O₂-Menge dem gewünschten Partialdruck von 3 bar entsprach. Der Autoklav wurde bis zu einer Reaktionstemperatur von 55-56 °C aufgeheizt, und nach einer Reaktionszeit von 18 Stunden wurde die Produktzusammensetzung mittels GC analysiert. Dabei wurde die Produktverteilung durch Integration der unkorrigierten Peakflächen im Gaschromatogramm ermittelt. Die Identifizierung der Oxidationsprodukte erfolgte mittels GC/MS-Analyse. Die Reaktionsergebnisse sind in Tabelle 2 zusammengestellt.

**Tabelle 2**

| Beispiel Nr. | Olefin | Umsatz (%)[a] | Epoxide | Selektivität (%)[b] |
|---|---|---|---|---|
| 6 | Cyclohexen | > 99 | Epoxycyclohexan 2,3-Epoxycyclohexan-1-on | 85 6 |
| 7 | trans-3-Hexen | 96 | 3,4-Epoxyhexan | > 98 |
| 8 | (R)-(+)-Limonen | 51 | cis-1,2-Epoxy-(R)-(+)-Limonen | 60 |
| | | | trans-1,2-Epoxy-(R)-(+)- | 31 |
| | | | Limonen 8,9-Epoxy-(R)-(+)-Limonen[c] | 9 |
| 9 | 1-Octen | 36 | 1,2-Epoxyoctan 3,4-Epoxyoctan | 87 5 |
| 10 | Isophoron | 16 | Epoxyisophoron | 30 |

| | | | | |
|---|---|---|---|---|
| [a] bezogen auf das Olefin | | | | |
| [b]Selektivität = (GC-Flächenanteil des Epoxides)/(GC-Flächenanteil aller detektierten Oxidationsprodukte des Olefins). Oxidationsprodukte mit einem Flächenanteil kleiner 0.2 % wurden vernachlässigt. | | | | |
| [c]2 Isomere. Die Konfigurationen konnten mittels GC/MS-Analyse nicht zugewiesen werden. | | | | |

### Beispiel 11

In einem 100 ml Stahlautoklaven wurden 750 µl (8.22 mmol) Isobutyraldehyd unter einer Propenatmosphäre (100 ml = 4.10 mmol) vorgelegt, und CO₂ wurde bis zu einer Dichte (ρ(CO₂) = Einwaage CO₂/Reaktorvolumen) von 0.75 g cm⁻³ mittels Kompressor zugefügt. Anschließend wurde der Druck im Autoklaven mit molekularem Sauerstoff so weit erhöht, daß die 02-Menge dem gewünschten Partialdruck von 5 bar entsprach. Der Autoklav wurde bis zu einer Reaktionstemperatur von 55-56 °C aufgeheizt, und nach einer Reaktionszeit von 18 Stunden über eine mit abs. Toluol gefüllte Kühlfalle mit Glasfritteneinsatz bei -50 °C entspannt. Der Reaktor wurde mit abs. Toluol ausgespült und die Toluol-Lösungen vereinigt. Die Produktzusammensetzung wurde mittels GC analysiert. Dabei wurde die Produktverteilung durch Integration der korrigierten Peakflächen im Gaschromatogramm ermittelt. Die Identifizierung der Oxidationsprodukte erfolgte mittels GC/MS-Analyse. Zur Bestimmung der Ausbeute an Oxidationsprodukten wurde n-Heptan als interner Standard verwendet. Der Umsatz an Propen war >73 % mit einer Selektivität bezüglich Epoxypropan von 14 %.

### Beispiel 12 - 15

In einem 100 ml Stahlautoklaven wurden 70 ml absolutes Toluol vorgelegt. Das Olefin (2.50 mmol) wurde zusammen mit dem Additiv Isobutyraldehyd (5.00 mmol) unter Argon zugegeben und der Gasraum des Autoklaven mit molekularem Sauerstoff gespült. An den Autoklavendeckel wurde eine mit Sauerstoff gefüllte Gasbürette angeschlossen. Es wurde 18 Stunden bei einer Reaktionstemperatur von 55-56 °C mit 850 U/min gerührt. Die Reaktionslösung wurde nach Abkühlung auf Raumtemperatur mittels GC analysiert. Dabei wurde die Produktverteilung durch Integration der Peakflächen im Gaschromatogramm ermittelt. Die Peakfläche von Epoxycyclooctan wurde durch Eichmessungen normiert, alle übrigen Daten sind unkorrigiert. Die Identifizierung der Oxidationsprodukte erfolgte mittels GC/MS-Analyse. Die Reaktionsergebnisse sind in Tabelle 3 zusammengestellt. Die Umsätze liegen unter ansonsten identischen Bedingungen in allen Fällen unter denen, die in CO₂ als Lösungsmittel erzielt wurden. Ferner wurden neben den angegebenen Reaktionsprodukten bei allen Reaktionen in Toluol Oxidationsprodukte des Toluols (Benzaldehyd, Benzylalkohol, Benzylhydroperoxid, Benzoesäure) nachgewiesen.

**Tabelle 3**

| Beispiel Nr. | Olefin | Umsatz (%)[a] | Epoxide | Selektivität (%)[b] |
|---|---|---|---|---|
| 12 | cis-Cycloocten | 84 | Epoxycyclooctan | > 99 |
| 13 | trans-3-Hexen | 69 | 3,4-Epoxyhexan | > 98 |
| 14 | 1-Octen | 23 | 1,2-Epoxyoctan 3,4-Epoxyoctan | 67 7 |
| 15 | Isophoron | 4 | Epoxyisophoron | 18 |

| | | | | |
|---|---|---|---|---|
| [a] bezogen auf das Olefin | | | | |
| [b] Selektivität = (GC-Flächenanteil des Epoxides)/(GC-Flächenanteil aller detektierten Oxidationsprodukte des Olefins). Oxidationsprodukte mit einem Flächenanteil kleiner 0.2 % wurden vernachlässigt. | | | | |

## Patentansprüche

1. Verfahren zur Herstellung von Epoxiden durch Oxidation von Olefinen mittels molekularem Sauerstoff unter Zusatz aldehydischer Additive, **dadurch gekennzeichnet, daß** die Oxidation in komprimiertem Kohlendioxid als Lösungsmittel durchgeführt wird.

2. Verfahren nach Anspruch 1, **dadurch gekennzeichnet, daß** als Olefine Verbindungen der allgemeinen Formel **I** 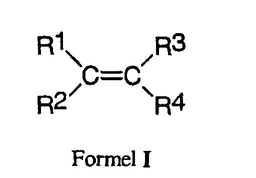 eingesetzt werden, wobei R¹, R², R³ und R⁴ gleich oder verschieden sind, unabhängig voneinander wählbar sind und ein Wasserstoffatom sind oder geradkettige, verzweigte, cyclische oder aromatische (C₁-C₂₀)-Kohlenwasserstoffe enthalten, in denen ein oder mehrere Kohlenstoffe durch Heteroatome ersetzt sein können oder/und die mit halogen-, hydroxy-, alkoxy-, phenoxy-, acyloxy-, acyl-, alkoxycarbonyl- oder phenoxycarbonyl-Gruppen substituiert sein können, wobei die Reste R¹, R², R³ oder R⁴ untereinander zu Ringen oder kondensierten Ringsystemen verbunden sein können.

3. Verfahren nach Anspruch 1-2, **dadurch gekennzeichnet, daß** als aldehydisches Additiv Verbindungen der allgemeinen Formel **III** 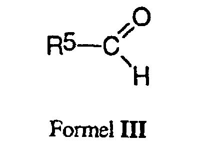 eingesetzt werden, wobei R⁵ ein Wasserstoffatom, eine (C₁-C₁₀)-Alkylgruppe, eine halogen-substituierte (C₁-C₁₀)-Alkylgruppe, eine Arylgruppe, eine halogen-substituierte Arylgruppe, eine Arylalkylgruppe oder eine halogen-substituierte Arylalkylgruppe ist.

4. Verfahren nach Anspruch 1-3, **dadurch gekennzeichnet, daß** der Gesamtdruck während der Reaktion im Bereich von 40 - 500 bar, bevorzugt von 60 - 200 bar liegt.

5. Verfahren nach Anspruch 1-4, **dadurch gekennzeichnet, daß** die Reaktionstemperatur im Bereich von 0 - 200 °C , bevorzugt von 31 - 100 °C liegt.

6. Verfahren nach Anspruch 4-5, wobei das komprimierte Kohlendioxid im überkritischen Zustand vorliegt.

7. Verfahren nach Anspruch 1-6, **dadurch gekennzeichnet, daß** der Partialdruch des molekularen Sauerstoffs im Bereich von 1 - 20 bar, bevorzugt 1 - 5 bar liegt.

8. Verfahren nach Anspruch 7, wobei der molekulare Sauerstoff in Form von reinem Sauerstoff eingesetzt wird.

9. Verfahren nach Anspruch 7, wobei der molekulare Sauerstoff in Form von Gemischen mit anderen Gasen eingesetzt wird.

10. Verfahren nach Anspruch 9, wobei der molekulare Sauerstoff in Form von Luftsauerstoff eingesetzt wird.

11. Verfahren nach Anspruch 1-10, **dadurch gekennzeichnet, daß** ein flüssiger, fester oder in der Reaktionsmischung gelöster und ansonsten bekannter Oxidationskatalysator zur Erhöhung der Reaktionsgeschwindigkeit oder Steigerung der Selektivität bei der Epoxidierung eingesetzt wird.

12. Verfahren nach Anspruch 1-11, **dadurch gekennzeichnet, daß** die Reaktionsmischung durch Stofftrennung mit komprimiertem CO₂ aufgearbeitet wird.

13. Verfahren nach Anspruch 12, wobei Reaktion und Stofftrennung in einem integrierten Verfahren kombiniert sind.

## Claims

1. A method for the preparation of epoxides by the oxidation of olefins using molecular oxygen with the addition of aldehyde additives, **characterized in that** the oxidation is performed in compressed carbon dioxide as the solvent.

2. The method according to claim 1, **characterized in that** compounds of general formula I 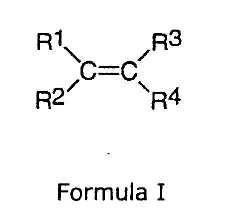 are employed as said olefins wherein R¹, R², R³ and R⁴ may be the same or different, can be independently selected and represent a hydrogen atom or straight or branched chain, cyclic or aromatic C₁-C₂₀ hydrocarbons in which one or more carbons may be replaced by heteroatoms or/and which may be substituted with halogen, hydroxy, alkoxy, phenoxy, acyloxy, acyl, alkoxycarbonyl or phenoxycarbonyl groups, wherein the residues R¹, R², R³ or R⁴ may be interconnected to form rings or fused ring systems.

3. The method according to claims 1-2, **characterized in that** compounds of general formula III 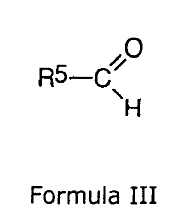 are employed as said aldehyde additive wherein R⁵ is a hydrogen atom, a C₁-C₁₀ alkyl group, a halogen-substituted C₁-C₁₀ alkyl group, an aryl group, a halogen-substituted aryl group, an arylalkyl group or a halogen-substituted arylalkyl group.

4. The method according to claims 1-3, **characterized in that** the total pressure during the reaction is within a range of from 40 to 500 bar, preferably from 60 to 200 bar.

5. The method according to claims 1-4, **characterized in that** the reaction temperature is within a range of from 0 to 200 °C, preferably from 31 to 100 °C.

6. The method according to claims 4-5, wherein said compressed carbon dioxide is in a supercritical state.

7. The method according to claims 1-6, **characterized in that** the partial pressure of the molecular oxygen is within a range of from 1 to 20 bar, preferably from 1 to 5 bar.

8. The method according to claim 7, wherein said molecular oxygen is employed in the form of pure oxygen.

9. The method according to claim 7, wherein said molecular oxygen is employed in the form of admixtures with other gases.

10. The method according to claim 9, wherein said molecular oxygen is employed in the form of atmospheric oxygen.

11. The method according to claims 1-10, **characterized in that** an oxidation catalyst, which may be liquid, solid or dissolved in the reaction mixture and which is otherwise known, is employed to increase the reaction rate or enhance selectivity in the epoxidation.

12. The method according to claims 1-11, **characterized in that** the reaction mixture is processed by a material separation in compressed CO₂.

13. The method according to claim 12, wherein said reaction and material separation are combined in one integrated process.

## Revendications

1. Procédé pour fabriquer des époxydes par oxydation d'oléfines à l'aide d'oxygène moléculaire, moyennant l'apport d'additifs à base d'aldéhyde, **caractérisé en ce que** l'oxydation est exécutée dans du dioxyde de carbone comprimé en tant que solvant.

2. Procédé selon la revendication 1, **caractérisé en ce qu'**on utilise comme oléfines, des composés répondant à la formule générale I 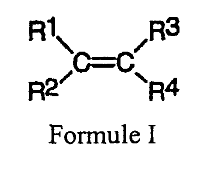 dans laquelle R¹, R², R³ et R⁴ sont identiques ou différents, peuvent être choisis indépendamment les uns des autres et représentent un atome d'hydrogène ou contiennent des hydrocarbures en (C₁-C₂₀) à chaîne linéaire, ramifiés, cycliques ou aromatiques, dans lesquels un ou plusieurs carbones peuvent être remplacés par des hétéroatomes et/ou qui peuvent être substitués par des groupes halogène, hydroxy, alcoxy, phénoxy, acyloxy, acyle, alcôxycarbonyle ou phénoxycarbonyle, les restes R¹ R², R³ ou R⁴ pouvant être reliés entre eux pour former des cycles ou des systèmes cycliques condensés.

3. Procédé selon l'une des revendications 1 et 2, **caractérisé en ce qu'**on utilise comme additif à base d'aldéhyde, des composés répondant à la formule générale III 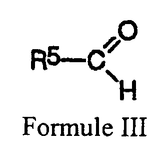 dans laquelle R⁵ représente un atome d'hydrogène, un groupe alkyle en (C₁-C₁₀), un groupe alkyle en (C₁-C₁₀) substitué par halogène, un groupe aryle, un groupe aryle substitué par halogène, un groupe arylalkyle ou un groupe arylalkyle substitué par halogène.

4. Procédé selon l'une des revendications 1 à 3, **caractérisé en ce que** la pression totale pendant la réaction se situe dans la gamme de 40 à 500 bars, de préférence de 60 à 200 bars.

5. Procédé selon l'une des revendications 1 à 4, **caractérisé en ce que** la température de réaction se situe dans la gamme de 0 à 200°C, de préférence de 31 à 100°C.

6. Procédé selon l'une des revendications 4 et 5, selon lequel le dioxyde de carbone comprimé est présent à l'état surcritique.

7. Procédé selon l'une des revendications 1 à 6, **caractérisé en ce que** la pression partielle de l'oxygène moléculaire se situe dans la gamme de 1 à 20 bars, de préférence de 1 à 5 bars.

8. Procédé selon la revendication 7, selon lequel l'oxygène moléculaire est utilisé sous la forme d'oxygène pur.

9. Procédé selon la revendication 7, selon lequel l'oxygène moléculaire est utilisé sous la forme de mélanges avec d'autres gaz.

10. Procédé selon la revendication 9, selon lequel l'oxygène moléculaire est utilisé sous la forme d'oxygène atmosphérique.

11. Procédé selon l'une des revendications 1 à 10, **caractérisé en ce qu'**on utilise un catalyseur d'oxydation liquide, solide ou dissous dans le mélange réactionnel et connu par ailleurs pour accroître la vitesse de réaction ou augmenter la sélectivité lors de l'époxydation.

12. Procédé selon l'une des revendications 1 à 11, **caractérisé en ce que** le mélange réactionnel est réutilisé par séparation des matières avec du CO₂ comprimé.

13. Procédé selon la revendication 12, selon lequel la réaction et la séparation de matières sont combinées dans un procédé intégré.
